# EUROPEAN PATENT SPECIFICATION

(11) **EP 2 679 689 B1**
(45) Date of publication and mention of the grant of the patent: **23.03.2016**
(21) Application number: 12173765.4
(22) Date of filing: 27.06.2012
(51) Int. Cl.: C12Q 1/68

(54) **Method for improved quantification of miRNAs**
Verfahren zur verbesserten Quantifizierung von miRNAs
Méthode pour une meilleure quantification des miRNAs

(43) Date of publication of application: 01.01.2014
(73) Proprietor: Siemens Aktiengesellschaft, 80333 München (DE); Siemens Healthcare Diagnostics Holding GmbH, 65760 Eschborn (DE)
(72) Inventor: Keller, Andreas, Dr., 66346 Püttlingen (DE); Kappel, Andreas, Dr., 61462 Koenigstein (DE); Stähler, Cord Friedrich, 69493 Hirschberg an der Bergstraße (DE)
(74) Representative: Maier, Daniel Oliver

(56) References cited:
- WO-A1-2010/085715
- WO-A1-2011/097528
- HANA SÍPOVÁ ET AL: "Surface Plasmon Resonance Biosensor for Rapid Label-Free Detection of Microribonucleic Acid at Subfemtomole Level", ANALYTICAL CHEMISTRY, vol. 82, no. 24, 15 December 2010 (2010-12-15), pages 10110-10115, XP55038008, ISSN: 0003-2700, DOI: 10.1021/ac102131s
- ABRAHAM J. QAVI ET AL: "Anti-DNA:RNA Antibodies and Silicon Photonic Microring Resonators: Increased Sensitivity for Multiplexed microRNA Detection", ANALYTICAL CHEMISTRY, vol. 83, no. 15, 1 August 2011 (2011-08-01), pages 5949-5956, XP55038012, ISSN: 0003-2700, DOI: 10.1021/ac201340s
- STOLLAR B D ET AL: "Immunochemical approaches to gene probe assays", ANALYTICAL BIOCHEMISTRY, ACADEMIC PRESS INC, NEW YORK, vol. 161, no. 2, 1 March 1987 (1987-03-01) , pages 387-394, XP024817717, ISSN: 0003-2697, DOI: 10.1016/0003-2697(87)90467-2 [retrieved on 1987-03-01]
- MATTHEWS J A ET AL: "Analytical strategies for the use of DNA probes", ANALYTICAL BIOCHEMISTRY, ACADEMIC PRESS INC, NEW YORK, vol. 169, no. 1, 15 February 1988 (1988-02-15), pages 1-25, XP024823244, ISSN: 0003-2697, DOI: 10.1016/0003-2697(88)90251-5 [retrieved on 1988-02-15]
- ZHAN ET AL: "MicroRNA expression dynamics during murine and human erythroid differentiation", EXPERIMENTAL HEMATOLOGY, ELSEVIER INC, US, vol. 35, no. 7, 19 June 2007 (2007-06-19), pages 1015-1025, XP022121914, ISSN: 0301-472X, DOI: 10.1016/J.EXPHEM.2007.03.014

### SEQUENCE LISTING

<110> Siemens AG
<120> Method for improved quantification of miRNAs
<130> Method for improved quantification of miRNAs
<160> 3
<170> BiSSAP 1.0
<210> 1
   <211> 22
   <212> RNA
   <213> Homo sapiens
<220>
   <221> source
   <222> 1..22
   <223> /mol_type="RNA"
   /note="hsa-miR-486-5p"
   /organism="Homo sapiens"
<400> 1
   uccuguacug agcugccccg ag 22
<210> 2
   <211> 22
   <212> RNA
   <213> Homo sapiens
<220>
   <221> source
   <222> 1..22
   <223> /mol_type="RNA"
   /note="hsa-miR-92a-3p"
   /organism="Homo sapiens"
<400> 2
   uauugcacuu gucccggccu gu 22
<210> 3
   <211> 22
   <212> RNA
   <213> Homo sapiens
<220>
   <221> source
   <222> 1..22
   <223> /mol_type="RNA"
   /note="hsa-miR-451a"
   /organism="Homo sapiens"
<400> 3
   aaaccguuac cauuacugag uu 22

## Description

### Field of the invention

The present invention relates to a method and kit for detecting a miRNA nucleic acid in a biological sample.

### Background of the invention

Very recently, molecular diagnostics has increasingly gained in importance. It has found an entry into the clinical diagnosis of diseases (inter alia detection of infectious pathogens, detection of mutations of the genome, detection of diseased cells and identification of risk factors for predisposition to a disease).

Today, specific nucleic acid sequences are typically detected or quantified by e.g. heterogenous hybridization assays, PCR methods, or direct sequencing. Those types of assays typically have a quite long time to result, which makes them difficult to apply for critical medical situations e.g. in the course of cardiac events. Moreover, the current tests are not adaptable to immunoassay analyzers, which are the most prominent platforms in the clinical laboratory or in point-of-care settings such as emergency rooms.

Nucleic acids of interest to be detected include genomic DNA, expressed mRNA and other RNAs such as MicroRNAs (abbreviated miRNAs). MiRNAs are a new class of small RNAs with various biological functions (A. Keller et al., Nat Methods. 2011 8(10):841-3). They are short (average of 20-24 nucleotide) ribonucleic acid (RNA) molecules found in eukaryotic cells. Several hundred different species of microRNAs (i.e. several hundred different sequences) have been identified in mammals. They are important for post-transcriptional gene-regulation and bind to complementary sequences on target messenger RNA transcripts (mRNAs), which can lead to translational repression or target degradation and gene silencing. As such they can also be used as biologic markers for research, diagnosis and therapy purposes.

Respective diagnostic tests typically quantify a panel of several miRNA species in a RNA preparation from tissue or ideally from blood. Today, several methodologies are applied, e.g. qRT-PCR or microarrays. Next generation sequencing (NGS) is an emerging methodology that allows the analysis and quantification of RNA species in an unbiased fashion, and with a superior dynamic range when compared to other methodologies.

The Document WO 2011097528 A1 discloses binding miRNA by using DNA probes hybridized to target RNA molecules and binding the hybrid nucleic acid duplex antibody specific for RNA/DNA hybrids.

However, out of all given miRNA species in a given sample, only certain select miRNA species are interesting as analyte (i.e. as nucleic acid of interest) for a given disease indication, while the majority of miRNA are not used for analysis. For example, a blood sample may contain hundreds or thousands of different miRNA species in different concentrations, while only a few (i.e.<50 or <10) species may actually be of interest for any given application, such as a diagnostic test. Especially when using NGS, a lot of sequence reads are thus "wasted" on irrelevant miRNA species, which increases time and cost of assay, as well as noise vs. signal.

There is thus a need for improved sample preparation or purification to allow more efficient analysis of those miRNA species which are of interest, e.g. which are disease specific.

### Definitions

Unless defined otherwise, technical and scientific terms used herein have the same meaning as commonly understood by one of ordinary skill in the art to which this invention belongs.

The term "antibody", as used herein, refers to an immunoglobulin protein or a fragment thereof, said fragment being capable of specifically binding an antigen. An antibody, including an antibody fragment, suitable for the invention may be monoclonal, polyclonal, of any host organism source, recombinantly expressed or otherwise artificially produced and of any immunoglobuline type. In the context of the invention, the antibody is able to bind an antigen comprising a hybrid of a nucleic acid molecule of interest and a nucleic acid probe.

The term "hybridization ", as used herein, refers to the process of combining complementary, single-stranded nucleic acids or nucleotide analogues into a single double stranded molecule, the so-called "hybrid". Nucleotides or nucleotide analogues will bind to their complement under normal conditions, so two complementary strands will bind to each other readily. Single stranded probes can be used in order to find complementary target sequences. If such sequences exist in the sample, the probes will hybridize to said sequences which can then be bound and in the bound state can e.g. be detected or removed from a sample.

The term "marker" or "biomarker" refers to a biological molecule, e.g., a nucleic acid, peptide, protein, hormone, etc., whose presence or concentration can be detected and correlated with a known condition, such as a disease state.

The term "nucleic acid" is intended to indicate any nucleic acid molecule and/or analogous molecules comprised of a sequence of nucleotides including DNA, cDNA and/or genomic DNA, RNA, preferably miRNA, peptide nucleic acid (PNA), locked nucleic acid (LNA) and/or morpholino.

"Nucleic acid of interest", within the meaning of the invention refers to a target nucleic acid which is to be detected and/or quantified. In particular, this term refers to a nucleic acid having a specific predetermined sequence.

"Nucleic acid probes", within the meaning of the invention, shall have the ordinary meaning of this term which is well known to the person skilled in the art of molecular biology. In a preferred embodiment of the invention they shall be understood as being polynucleotide molecules having a sequence identical, complementary, or homologous to the complement of regions of a target nucleic acid of interest which is to be detected or quantified. In yet another embodiment, nucleotide analogues are also comprised for usage as nucleic acid probes.

The terms "sample", "biological sample", or "clinical sample", as used herein, refer to any sample containing a nucleic acid of interest. A sample may be obtained from a patient. The sample may be of any biological tissue or fluid. Such samples include, but are not limited to, sputum, blood, serum, plasma, blood cells (e.g., white cells), tissue, biopsy samples, smear samples, lavage samples, swab samples, cell-containing body fluids, free floating nucleic acids, urine, peritoneal fluid, and pleural fluid, liquor cerebrospinalis, urine, feces, tear fluid, or cells therefrom. Biological samples may also include sections of tissues such as frozen or fixed sections taken for histological purposes or microdissected cells or extracellular parts thereof.

The term "solid phase", as used herein, refer to an object which consists of a porous and/or nonporous, material which is insoluble in the solvent system used (e.g. water insoluble). It can have a wide variety of forms such as vessels, tubes, plates, spheres, microparticles, rods, strips, filter paper, chromatography paper, etc. A wide variety of materials can be used as solid phase, including plastic materials, natural polymers, such as cellulose or cellulose derivatives, metal materials such as gold, glass materials, or ceramic materials. The term "solid phase bound" refers to an object, such as a nucleic acid probe or an antibody, being attached to the solid phase in such a way that it will not become detached and dissolved into solution when the methods of the invention described herein are performed. Binding may be achieved for example by physissorbtion, chemisorption, covalent linking, as is known in the art.

The term "array" refers to an arrangement of addressable locations on a device, e.g. a chip device. The number of locations can range from several to at least hundreds or thousands. Each location represents an independent reaction site. Arrays include protein arrays and antibody-arrays. A "nucleic acid array" refers to an array containing nucleic acid probes, such as oligonucleotides, polynucleotides or larger portions of genes. The nucleic acid on the array is preferably single stranded. A "microarray" refers to a biochip or biological chip, i.e. an array of regions having a density of discrete regions with immobilized probes of at least about 100/cm2.

A "PCR-based method" refers to methods comprising a polymerase chain reaction PCR. This is a method of exponentially amplifying nucleic acids, e.g. DNA or RNA by enzymatic replication in vitro using one, two or more primers. For RNA amplification, a reverse transcription may be used as a first step. PCR-based methods comprise kinetic or quantitative PCR (qPCR) which is particularly suited for the analysis of expression levels,). When it comes to the determination of expression levels, a PCR based method may for example be used to detect the presence of a given RNA molecule by (1) reverse transcription of the complete RNA pool (the so called transcriptome) into cDNA with help of a reverse transcriptase enzyme, and (2) detecting the presence of a given cDNA with help of respective primers. This approach is commonly known as reverse transcriptase PCR (rtPCR). The term "PCR based method" comprises both end-point PCR applications as well as kinetic/real time PCR techniques applying special fluorophors or intercalating dyes which emit fluorescent signals as a function of amplified target and allow monitoring and quantification of the target. Quantification methods could be either absolute by external standard curves or relative to a comparative internal standard.

The term "next generation sequencing" or "high throughput sequencing" refers to high-throughput sequencing technologies that parallelize the sequencing process, producing thousands or millions of sequences at once. Examples include Massively Parallel Signature Sequencing (MPSS) Polony sequencing, 454 pyrosequencing, Illumina (Solexa) sequencing, SOLiD sequencing, Ion semiconductor sequencing, DNA nanoball sequencing, Helioscope(TM) single molecule sequencing, Single Molecule SMRT(TM) sequencing, Single Molecule real time (RNAP) sequencing, Nanopore DNA sequencing.

### Object of the invention

The technical problem underlying the present invention is to provide a method for preparing a sample for the detection of miRNA nucleic acids molecules of interest which is efficient and easily adaptable to automated laboratory platforms or point-of care type settings.

It is a further object of the invention to provide a method for preparing a sample for the detection of miRNA nucleic acids molecules of interest which allows to obtain quantifiable results with an improved dynamic range.

It is another object of the present invention to provide a kit for preparing a sample for the detection of miRNA nucleic acids molecules of interest meeting the above criteria.

These objects are met with the methods and means according to the independent claims of the present invention. The dependent claims are related to preferred embodiments.

### Summary of the invention

Before the invention is described in detail, it is to be understood that this invention is not limited to the particular component parts of the process steps of the methods described as such methods may vary. It is also to be understood that the terminology used herein is for purposes of describing particular embodiments only, and is not intended to be limiting. It must be noted that, as used in the specification and the appended claims, the singular forms "a," "an" and "the" include singular and/or plural referents unless the context clearly dictates otherwise. It is also to be understood that plural forms include singular and/or plural referents unless the context clearly dictates otherwise. It is moreover to be understood that, in case parameter ranges are given which are delimited by numeric values, the ranges are deemed to include these limitation values.

In its most general term, the invention relates to a method of enriching miRNA nucleic acids molecules of interest in a sampe by selective removal of nucleic acid species, in particular miRNA species, which are not of interest. The invention is based on the surprizing and previously unknown information that a single miRNA species or only a few individual miRNA species make up the majority of all miRNA species in a sample. Selective removal of these species prior to detection of miRNA nucleic acids molecules will improve assay time, assay sensitivity, and reproducibility.

The method and kit of the invention allow a fast detection and quantification of specific miRNA nucleic acids molecules of interest.

In particular, the invention relates to the method according to claiml. .

In one aspect, the invention relates to a method for preparing a sample for detecting a miRNA nucleic acid molecule of interest in said sample, comprising the following step:
- selectively removing at least one miRNA species, said at least one abundant miRNA species having a relative abundance of at least 1%.

The term "relative abundance" means the number of molecules of the at least one abundant miRNA species relative to the number of molecules of all miRNA species in the sample. An miRNA species is abundant, if it has a relative abundance of at least 1%, 5%, 10%, 20%, or 50% in a given sample.

The term "selectively removing" refers to any process which allows to remove an abundant miRNA species from a sample while not removing another miRNA species, in particular an miRNA of interest.

In this regard, "preparing a sample" refers to a method of purification before the actual miRNA of interest is detected.

The detection of the miRNA of interest can then be qualitative or quantitative.

According to the invention, the invention relates to a method for preparing a sample for detecting a miRNA nucleic acid molecule of interest in the sample, comprising the following step:
- selectively removing at least one of the abundant miRNA species selected from the group consisting of hsa-miR-486-5p, hsa-miR-92a-3p and hsa-miR-451a.

According to an aspect of the invention said at least one miRNA species is hsa-miR-486-5p.

Following the step of selectively removing the at least one miRNA selected from hsa-miR-486-5p, hsa-miR-92a-3p and hsa-miR-451a, a step of detecting the miRNA nucleic acid molecule of interest may ensue. The detection of the miRNA may be accomplished by any appropriate detection method, such as immunoassay, polymerase chain reaction, hybridization with a labelled probe, microarray, sequencing and others. A preferred method is next generation sequencing.

According to an aspect of the invention the step of at selectively removing at least one of the miRNA species comprises selectively binding said miRNA species with a nucleic acid probe.

According to an aspect of the invention the nucleic acid probe is a DNA probe.

According to an aspect of the invention the nucleic acid probe is bound to a solid phase.Further, according to this aspect he duplex of DNA probe and the at least one miRNA can be bound by a anti-DNA:RNA antibody.

According to an aspect of the invention the nucleic acid anti-DNA:RNA antibody is bound to a solid phase.

According to an aspect of the invention the solid phase is selected from the group consisting of plastic material, bead, magnetic bead, and filter material.

According to this further aspect of the invention, the solid phase can have a very wide variety of forms, for example those of vessels, tubes, microtitration plates, spheres, microparticles, , magnetic particles, rods, strips, filter paper, chromatography paper, etc. As a rule, the surface of the solid phase is hydrophilic or can be made hydrophilic. The solid phase can consist of a very wide variety of materials, for example of inorganic and/or organic materials, of synthetic materials, of naturally occurring materials and/or of modified naturally occurring materials. Examples of solid phase materials are polymers, such as cellulose, nitrocellulose, cellulose acetate, polyvinyl chloride, polyacrylamide, crosslinked dextran molecules, agarose, polystyrene, polyethylene, polypropylene, polymethacrylate or nylon; ceramic, glass or metals, in particular precious metals such as gold and silver; magnetite; mixtures or combinations thereof; etc..

The solid phase can also possess a coating consisting of one or more layers, for example of proteins, carbohydrates, lipophilic substances, biopolymers or organic polymers, or mixtures thereof, in order, for example, to suppress or prevent the nonspecific binding of sample constituents to the solid phase or in order, for example, to achieve improvements with regard to the suspension stability of particular solid phases, with regard to storage stability, with regard to dimensional stability or with regard to resistance to UV light, microbes or other agents having a destructive effect.

According to an aspect of the invention the duplex of DNA probe and the at least one miRNA is brought into contact with a nuclease that degrades DNA:RNA hybrids. This allows selective degradation of the duplex bound of the at least one miRNA hsa-miR-486-5p, hsa-miR-92a-3p or hsa-miR-451a, while the miRNA nucleic acid of interest remains intact.

According to an aspect of the invention all three the miRNA species hsa-miR-486-5p, hsa-miR-92a-3p and hsa-miR-451a are selectively removed from the sample.

According to an aspect of the invention the sample is selected from the group consisting of blood sample, plasma sample and serum sample.

Herein further disclosed is a kit for detecting a nucleic acid molecule of interest in a sample, said kit being useful for carrying out a method of any one of the aforementioned claims, comprising at least one nucleic acid probe capable of selectively binding at least one abundant miRNA species, preferably at least one of the miRNA species selected from the group consisting of hsa-miR-486-5p, hsa-miR-92a-3p and hsa-miR-451a.

It is disclosed that the nucleic acid probe of the kit is bound to a solid phase.

It is disclosed that the kit comprises three nucleic acid probes capable of selectively binding the miRNA species hsa-miR-486-5p, hsa-miR-92a-3p and hsa-miR-451a respectively.

It is disclosed that the nucleic acid probe is labelled with a binding moiety which can be bound by a binding partner. According to a preferred embodiment the binding moiety is provided by biotinylation of the nucleic acid probe. This allows binding by streptavidin.

The binding partner can be provided on a solid phase. Such a kit is easily adaptable to any abundant miRNA species by adapting the nucleic acid probe accordingly.

It is disclosed that the detection is a quantitative detection. If desired, the concentration can be measured as an absolute concentration, for example, by using an internal standard (a known nucleic acid with a known initial concentration, which is added to the sample or is amplified and measured in parallel). In many cases it may be sufficient to determine the relative concentration of the nucleic acid of interest in the test sample against a reference sample, e.g. by comparing a patient sample against a sample from a healthy control.

In the kit, the nucleic acid probe can be provided in a solution, either as a working solution or as a dilutable stock solution. Alternatively it can be provided in a dissolvable form, for example in lyophilized form.

The kit can further comprise devices and/or reagents for preparation of total RNA.

It is disclosed that the kit may comprise the probe in a solid phase-bound form on pre-coated microtiter plates, coated beads, test strips and the like. For example, the solid phase, such as a microtiter plate (e.g. a 96-well plate), can be pre-coated with the nucleic acid probe for binding at least one of the miRNA species selected from the group consisting of hsa-miR-486-5p, hsa-miR-92a-3p and hsa-miR-451a. Alternatively, the solid phase can be coated with a anti-DNA:RNA antibody and the nucleic acid probe or probes for for binding at least one of the miRNA species selected from the group consisting of hsa-miR-486-5p, hsa-miR-92a-3p and hsa-miR-451a can be provided in solution or in dissolvable form.

### Brief description of the examples

Additional details, features, characteristics and advantages of the object of the invention are further disclosed in the following description of the respective examples, which, in an exemplary fashion, show preferred embodiments of the present invention. However, these examples should by no means be understood as to limit the scope of the invention.

The invention is based on the surprizing and previously unknown information that only a few abundant individual miRNA species can make up a large proportion of all miRNA species in a sample.

Generally speaking, abundant miRNAs for selective removal can be e.g. identified as follows:
- obtain a plurality of samples of a given sample type (i.e. blood, serum, plasma, tissue, etc.) from healthy donors and patients with different diseases and disease states,
- prepare total RNA from said samples,
- detect a plurality of miRNAs in said samples and determine their relative abundance, and
- identify those miRNA having high abundance in samples from both healthy donors and patients, these are the high abundance miRNA which are not disease specific and can thus be selectively removed from the sample while the information content of disease specific marker miRNAs in the sample is maintained.

A preferred way to detect the plurality of miRNAs in said sample is by NGS sequencing as this is an unbiased method giving both qualitative (sequence information) and quantitative (i.e. number of reads) information.

The three miRNAs (hsa-miR-486-5p, hsa-miR-92a-3p and hsa-miR-451a) found to make up about 95% of all miRNA species in the tested samples are:
hsa-miR-486-5p (SEQ ID NO: 1)
uccuguacugagcugccccgag
hsa-miR-92a-3p (SEQ ID NO: 2)
uauugcacuugucccggccugu
hsa-miR-451a (SEQ ID NO: 3)
aaaccguuaccauuacugaguu

By removing at least one, preferably by removing at least hsa-miR-486-5p, more preferably by removing hsa-miR-486-5p and hsa-miR-92a-3p and most preferably by removing all three of hsa-miR-486-5p, hsa-miR-92a-3p and hsa-miR-451a, assay time and accuracy of a subsequent miRNA detection assay can be dramatically improved. E.g., the assay time of an NGS-based blood miRNA test can be dramatically improved by selectively removing the 3 most abundant miRNA species species (hsa-miR-486-5p, hsa-miR-92a-3p and hsa-miR-451a) from the RNA preparation by e.g. the following procedures prior to the NGS test:
1. (Magnetic) beads decorated with oligonucleotides that are complementary to hsa-miR-486-5p, hsa-miR-92a-3p and/or hsa-miR-451a are incubated with the sample, and are removed after the complementary miRNA molecules have bound.
2. The RNA sample is hybridized to DNA oligonucleotides that are complementary to hsa-miR-486-5p, hsa-miR-92a-3p and/or hsa-miR-451a. The sample is subsequently incubated with a surface-coated antibody that binds to DNA:RNA hybrids, th remove these three miRNA species from the sample.
3. The RNA sample is hybridized to DNA oligonucleotides that are complementary to hsa-miR-486-5p, hsa-miR-92a-3p and/or hsa-miR-451a. The sample is subsequently incubated with a nuclease that degrades DNA:RNA hybrids.

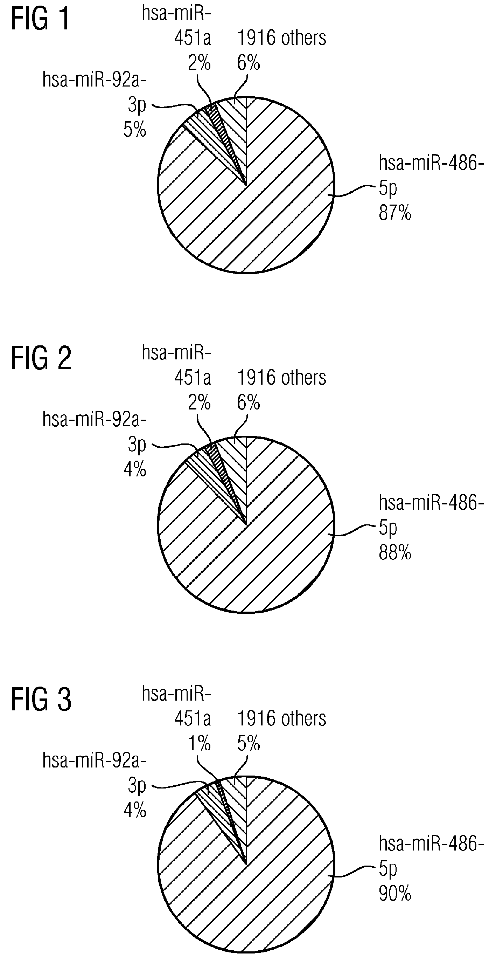

In this regard, reference is made to the figures which show data from blood RNA preparations
- from apparently healthy individuals (Figure 1),
- from patients with Alzheimer disease (Figure 2)
- from or patients with Multiple Sclerosis (Figure 3).

In the assays shown in Fis. 1-3, 1919 different miRNA transcripts were detected and quantified from blood samples by NGS. In each of these groups the three miRNAs (hsa-miR-486-5p, hsa-miR-92a-3p and hsa-miR-451a) constitute about 94-95% of all blood-bourne miRNA transkripts.

When using NGS for detecting miRNAs of interest, these three highly abundant miRNA species are therefore detected in about 19 out of 20 reads and block valuable assay time for detection of the remaining ca. 2000 low-abundant miRNA species. Interestingly, close to 90% of the transcripts are made up of hsa-miR-486-5p alone, so just the removal of this single one species of miRNA already allows for a very significant improvement in detection of a miRNA of interest.

As the majority of diagnostically relevant miRNA species belongs to the low-abundant fraction, a NGS test that selectively enriches the low-abundant miRNA species will be much faster than a test that quantifies all miRNA species.

Also in using other detection methods, such as immunoassay, hybridization based methods etc., removal of the three highly abundant miRNA species will result in greater assay sensitivity, less background signal, and greater dynamic range.

The sequences for the nucleic acid probes and miRNAs are given in the table 1 below:
To prepare samples, total RNA including miRNA can be isolated using a commercially available product for RNA isolateion, such as the PAXgene Blood miRNA Kit (Qiagen) following the manufacturers recommendations. Isolated RNA can be stored at -80°C. Samples can be prepared for detection of miRNAs of interest by incubating with probe-coated magnetic beads for removal of hsa-miR-486-5p, hsa-miR-92a-3p and hsa-miR-451a. After separating the beads from the sample (e.g. by magnetic separation), the miRNA of interest can be detected in the prepared sample.

## Claims

1. A method for preparing a sample for detecting a miRNA nucleic acid molecule of interest in said sample, comprising the following step:
- selectively removing at least one abundant miRNA species from the sample, said at least one miRNA species having a relative abundance of at least 1%, wherein said at least one miRNA species is selected from the group consisting of hsa-miR-486-5p, hsa-miR-92a-3p and hsa-miR-451a.

2. The method according to claim 1, wherein the step of at selectively removing at least one of the miRNA species comprises selectively binding said miRNA species with a nucleic acid probe.

3. The method according to claim 2 wherein the nucleic acid probe is a DNA probe.

4. The method according to claim 2 or 3 wherein the nucleic acid probe is bound to a solid phase.

5. The method according to claim 2, wherein the duplex of DNA probe and the at least one miRNA is bound by a anti-DNA:RNA antibody.

6. The method according to claim 5, wherein the nucleic acid anti-DNA:RNA antibody is bound to a solid phase.

7. The method according to any of claims 3 or 5 wherein the solid phase is selected from the group consisting of plastic material, bead, magnetic bead, and filter material.

8. The method according to claim 2, wherein the duplex of DNA probe and the at least one miRNA is brought into contact with a nuclease that degrades DNA:RNA hybrids.

9. The method according to any one of the aforementioned claims, wherein all three the miRNA species selected from the group consisting of hsa-miR-486-5p, hsa-miR-92a-3p and hsa-miR-451a are selectively removed.

10. The method according to any one of the aforementioned claims, wherein the sample is selected from the group consisting of blood sample, plasma sample and serum sample.

11. The method according to any one of the aforementioned claims, further comprising a step of detecting an miRNA of interest after the step of selectively removing the least one abundant miRNA species having a relative abundance of at least 1%.

12. The method of claim 11, wherein the step of detecting the miRNA of interest is performed by a method selected from a hybridization based method, a PCR based method, an NGS method, an array based method and combinations thereof.

## Patentansprüche

1. Verfahren zur Vorbereitung einer Probe für den Nachweis eines interessierenden miRNA-Nukleinsäuremoleküls in der Probe, das den folgenden Schritt umfasst:
- selektives Entfernen wenigstens einer häufigen miRNA-Spezies aus der Probe, wobei die wenigstens eine miRNA-Spezies eine relative Häufigkeit von wenigstens 1% aufweist, wobei die wenigstens eine miRNA-Spezies aus der aus hsa-miR-486-5p, hsamiR-92a-3p und hsa-miR-451a bestehenden Gruppe ausgewählt ist.

2. Verfahren nach Anspruch 1, wobei der Schritt des selektiven Entfernens der wenigstens einen miRNA-Spezies selektives Binden der miRNA-Spezies mit einer Nukleinsäuresonde umfasst.

3. Verfahren nach Anspruch 2, wobei es sich bei der Nukleinsäuresonde um eine DNA-Sonde handelt.

4. Verfahren nach Anspruch 2 oder 3, wobei die Nukleinsäuresonde an eine Festphase gebunden ist.

5. Verfahren nach Anspruch 2, wobei der Duplex von DNA-Sonde und der wenigstens einen miRNA durch einen Anti-DNA:RNA-Antikörper gebunden ist.

6. Verfahren nach Anspruch 5, wobei der Nukleinsäure-Anti-DNA:RNA-Antikörper an eine Festphase gebunden ist.

7. Verfahren nach einem der Ansprüche 3 oder 5, wobei die Festphase aus der aus Kunststoffmaterial, Kügelchen, Magnetkügelchen und Filtermaterial bestehenden Gruppe ausgewählt ist.

8. Verfahren nach Anspruch 2, wobei der Duplex von DNA-Sonde und der wenigstens einen miRNA mit einer Nuklease in Kontakt gebracht wird, die DNA:RNA-Hybride abbaut.

9. Verfahren nach einem der vorstehend genannten Ansprüche, wobei alle drei der aus der aus hsa-miR-486-5p, hsa-miR-92a-3p und hsa-miR-451a bestehenden Gruppe ausgewählten miRNA-Spezies selektiv entfernt werden.

10. Verfahren nach einem der vorstehend genannten Ansprüche, wobei die Probe aus der aus Blutprobe, Plasmaprobe und Serumprobe bestehenden Gruppe ausgewählt ist.

11. Verfahren nach einem der vorstehend genannten Ansprüche, das ferner einen Schritt des Nachweisens einer interessierenden miRNA nach dem Schritt des selektiven Entfernens der wenigstens einen häufigen miRNA-Spezies, die eine relative Häufigkeit von wenigstens 1% aufweist, umfasst.

12. Verfahren nach Anspruch 11, wobei der Schritt des Nachweisens der interessierenden miRNA mit einem aus einem auf Hybridisierung beruhenden Verfahren, einem auf PCR beruhenden Verfahren, einem NGS-Verfahren, einem Verfahren auf Array-Basis und Kombinationen davon ausgewählten Verfahren durchgeführt wird.

## Revendications

1. Procédé de préparation d'un échantillon pour détecter une molécule d'acide nucléique miARN de l'échantillon, à laquelle on s'intéresse, comprenant le stade suivant :
- on élimine sélectivement au moins une espèce miARN abondante de l'échantillon, la au moins une espèce miARN ayant une abondance relative d'au moins 1 %, la au moins une espèce miARN étant choisie dans le groupe consistant en hsa-miR-486-5p, hsa-mIR-92a-3p et hsa-mIR-451a.

2. Procédé suivant la revendication 1, dans lequel le stade d'élimination sélective d'au moins l'une des espèces miARN comprend fixer sélectivement l'espèce miARN par une sonde d'acide nucléique.

3. Procédé suivant la revendication 2,
dans lequel la sonde d'acide nucléique est une sonde d'ADN.

4. Procédé suivant la revendication 2 ou 3,
dans lequel la sonde d'acide nucléique est fixée à une phase solide.

5. Procédé suivant la revendication 2, dans lequel le duplex de la sonde d'ADN et le au moins un miARN est fixé par un anticorps anti-ADN:ARN.

6. Procédé suivant la revendication 5, dans lequel l'anticorps d'acide nucléique anti-ADN:ARN est fixé à une phase solide.

7. Procédé suivant l'une quelconque des revendications 3 ou 5, dans lequel la phase solide est choisie dans le groupe consistant en une matière plastique, une perle, une perle magnétique et une matière filtrante.

8. Procédé suivant la revendication 2, dans lequel le duplex de la sonde d'ADN est de au moins un miARN et mise en contact avec une nucléase qui dégrade l'hybride ADN:ARN.

9. Procédé suivant l'une quelconque des revendications précédentes, dans lequel on élimine sélectivement toutes les trois espèces miARN choisies dans le groupe consistant en hsamiR-486-5p, hsa-miR-92a-3p et hsa-miR-451a.

10. Procédé suivant l'une quelconque des revendications précédentes, dans lequel on choisit l'échantillon dans le groupe consistant en un échantillon de sang, un échantillon de plasma et un échantillon de sérum.

11. Procédé suivant l'une quelconque des revendications précédentes, comprenant en outre un stade de détection d'un miARN auquel on s'intéresse après le stade d'élimination sélective de la au moins une espèce abondante de miARN ayant une abondance relative d'au moins 1 %.

12. Procédé suivant la revendication 11, dans lequel on effectue le stade de détection du miARN auquel on s'intéresse par un procédé choisi parmi un procédé à base d'hybridation, un procédé à base de PCR, un procédé NGS, un procédé à base de matrice et leurs combinaisons.
